# FASCICULE DE BREVET EUROPEEN

(11) **EP 3 237 393 B1**
(45) Date de publication et mention de la délivrance du brevet: **26.09.2018**
(21) Numéro de dépôt: 15817875.6
(22) Date de dépôt: 24.12.2015
(51) Int. Cl.: C07D 317/22, A61K 31/341, A61P 25/00

(54) **COMPOSITION PRO-PHEROMONALE APAISANTE POUR LES MAMMIFERES**
LINDERNDE PROPHEROMONALE ZUSAMMENSETZUNG FÜR SÄUGETIERE
SOOTHING PRO-PHEROMONAL COMPOSITION FOR MAMMALS

(30) Priorité: 26.12.2014 FR 1463359
(43) Date de publication de la demande: 01.11.2017
(73) Titulaire: Melchior Material And Life Science France, 64170 LACQ (FR)
(72) Inventeur: GUERRET, Olivier, 46170 Pern (FR); GUILLONNEAU, Loic, 64000 Pau (FR); DUFOUR, Samuel, 64300 Orthez (FR)
(74) Mandataire: Regimbeau
(86) Numéro de dépôt international: PCT/EP2015/081241
(87) Numéro de publication internationale: WO 2016/102706

(56) Documents cités:
- US-A1- 2013 210 927
- CHARRA R ET AL: "Brain processing of the mammary pheromone in newborn rabbits", BEHAVIOURAL BRAIN RESEARCH, ELSEVIER, AMSTERDAM, NL, vol. 226, no. 1, 4 septembre 2011 (2011-09-04), pages 179-188, XP028319757, ISSN: 0166-4328, DOI: 10.1016/J.BBR.2011.09.008 [extrait le 2011-09-12]

## Description

### RESUME

La présente invention a pour objet un composé de formule générale (1) (en configuration Z ou E) ainsi que des compositions et formulations pro-phéromonales comprenant ledit composé. L'avantage de telles compositions est de permettre un relargage et une diffusion contrôlés et prolongés du 2-méthyl-2-buténal ainsi que du ou des acides gras ajouté aux compositions et formulations.

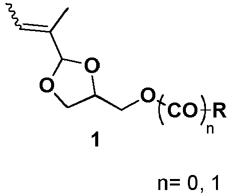

### CONTEXTE

Dans son interaction avec les animaux domestiques, qu'ils soient des animaux de compagnie ou des animaux d'élevage, l'homme a intérêt à ce que ces derniers aient un comportement calme et apaisé. Dans le cas particulier des cochons, ces animaux sont connus pour développer en situation normale mais surtout en situation de stress un comportement agressif. Ce comportement agressif se traduit notamment par des combats entre porcs ou même entre porcelets et par des tentatives de s'échapper (Mc Glone J.J., J. Animal Sc., 1990, volume 68, 86-97). Afin de réduire ce comportement et de permettre un meilleur engraissement des porcs d'élevage, de nombreux traitements neuroleptiques ont été testés et ont démontré leur efficacité (voir par exemple la revue de Dantzer R, Vet. Comm., 1977, volume 1, 161-177). Mais les effets indirects à long terme sur les individus qui consomment la viande des porcs traités ainsi restaient méconnus et suscitaient suffisamment d'inquiétude pour que d'autres solutions soient envisagées. En conséquence, depuis de nombreuses années, diverses solutions ont été proposées pour les divers types d'animaux domestiques et notamment l'utilisation de compositions contenant des phéromones.

Afin de mieux interagir avec l'animal, des compositions à base de phéromones produites par l'espèce en question ont d'abord été développées. Par exemple pour calmer les chiens, une composition contenant une phéromone artificielle apaisante copiant la phéromone naturelle produite par la chienne allaitante pour rassurer ses petits, a montré son efficacité (brevet WO2009134958). De la même manière, pour résoudre les problèmes de marquage des territoires des chats (urine, griffures...), une composition associant un ensemble d'acides gras présents dans les phéromones faciales naturelles du chat et des substances végétales apaisantes ou attractantes (extrait de valériane) a été mise au point (EP0724832). D'autres compositions anti-stress ont aussi prouvé leur efficacité telles que des compositions à base de squalène, d'acide linoléique et de docosanol pour les chiens (WO2011084795), des compositions à base d'un ratio spécifique d'acide linoléique et linolénique (58%/13%) avec ajout d'un flavonoïde (type gingembre) et éventuellement d'autres ajouts (houblon et tryptophane connu pour son effet sédatif) (US6576252), ou bien des compositions comportant au moins trois des acides gras présents dans les glandes mammaires des femelles dont l'acide palmitique, l'acide caprique, l'acide laurique, l'acide oléique, l'acide myristique, et l'acide linoléique. Ces dernières compositions se sont révélées efficaces pour divers mammifères en particulier les porcelets (US 6077867).

Cette première série de travaux a été complétée par une autre approche qui consiste à identifier des substances issues de glandes mammaires d'une autre espèce que celle qu'il s'agit d'apaiser (par exemple le lapin) et d'étudier comment elle affecte le comportement d'une autre espèce. Ainsi, il a été montré que, la phéromone maternelle du lapin, le 2-méthyl-2-buténal, (voir les travaux de l'équipe de Coureaud cités plus loin ou la revue de B. Schaal, Vitamins and Hormones, Volume 83, 2010, ch. IV, p. 83) qui est émise pour guider les lapereaux aveugles vers les mamelles de la lapine jusqu'à leur sevrage, a un effet sur les pulsations cardiaques du chien et sur son comportement (brevet US20140603). Cependant, ces différents travaux montrent que la durée d'efficacité du lait de lapin est courte (moins de 3 heures) et que les mélanges contenant le 2-méthyl-2-buténal présentent une efficacité très réduite dans le temps lorsqu'ils sont diffusés par des diffuseurs thermiques (brevet US20140603).

Il n'est pas surprenant que de tels problèmes existent puisque le point d'ébullition du 2-méthyl-2-buténal est de 116°C à pression atmosphérique tandis que les acides gras ou leurs dérivés ont des points d'ébullition supérieurs à 200°C sous de faibles pressions atmosphériques (l'acide linoléique a par exemple un point d'ébullition de 230°C sous 16 mmHg). Or, si le 2-méthyl-2-buténal est efficace à très faible dose (entre 10⁻⁵ et 10⁻⁹ g/l selon les publications de Coureaud : Coureaud G. et al, Chem. Sens. Volume 29, 2004, 341-350), les acides gras sont généralement utilisés à des concentrations plus importantes de l'ordre de 10⁻¹-10⁻² g/l correspondant à des taux journaliers supérieurs à 10 mg/jour et jusqu'à 200mg/jour (brevet WO2009144321). Pour que de tels ordres de grandeurs soient respectés, il faudrait que les volatilités relatives des composés soient inverses de ce qu'elles sont.

### INVENTION

L'objet de l'invention est donc une formulation permettant de libérer lentement et à des doses inférieures à 10⁻⁷ g/jour le 2-méthyl-2-buténal, tandis que les acides gras sont libérés à des taux journaliers supérieurs à 10⁻²-10⁻³ g/jour. Afin d'atteindre ce but, la demanderesse a eu le mérite de synthétiser un nouveau composé de formule générale (1). Lors de la diffusion d'une composition ou formulation contenant un ou des acides gras associés à ce composé (1), le 2-méthyl-2-buténal sera produit « in-situ » par décomposition du composé (1) et donc son relargage simultané avec celui des acides gras mieux contrôlé.

A cet effet, la présente invention concerne, dans un premier mode de réalisation, un composé de formule générale (1) : 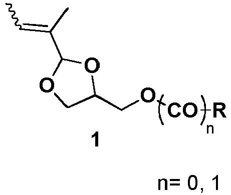 dans laquelle n est égal à 0 ou 1 et R représente un groupe alkyl linéaire saturé ou insaturé comportant de 2 à 30 atomes de carbone.

Dans un deuxième mode de réalisation de la présente invention, le composé de formule générale (1) est caractérisé en ce que n=0 et R est un groupe alkyl linéaire saturé ou insaturé comportant entre 9 et 30 atomes de carbone, plus particulièrement entre 12 et 30 atomes de carbone.

Dans un troisième mode de réalisation de l'invention, le composé (1) est caractérisé en ce que n=1 et R est un groupe alkyl linéaire saturé ou insaturé comportant de 2 à 30 atomes de carbone, plus particulièrement de 8 à 22 atomes de carbone.

Dans un mode de réalisation de l'invention, le groupe est R insaturé, en particulier monoinsaturé. R peut aussi être di-insaturé.
Dans un mode de réalisation préféré, le composé (1) est sous la forme Z.

Selon un quatrième mode de réalisation, la présente invention concerne un composé (1) selon l'un des modes de réalisation 1 à 3 pour son utilisation en tant que médicament vétérinaire pour le traitement de l'anxiété, du stress ou de l'agressivité chez un mammifère non humain.

Selon un cinquième mode de réalisation, la présente invention concerne un composé (1) selon le quatrième mode de réalisation, caractérisé en ce que le mammifère non humain est choisi dans le groupe comprenant les porcins, les ovins, les équidés, les bovins, les félins et les canidés.

Dans un mode de réalisation particulier, le mammifère non humain est le porc.

Dans un mode de réalisation particulier, le mammifère non humain est le chien.

Dans un mode de réalisation particulier, le mammifère non humain est le chat.

Dans un mode de réalisation particulier, le mammifère non humain est la vache.

Dans un sixième mode de réalisation, l'invention concerne une composition vétérinaire comprenant un composé (1) selon l'un des modes de réalisation 1 à 3 et au moins un acide gras ou un de ses dérivés.

La présente invention, dans un septième mode de réalisation concerne une composition selon le sixième mode de réalisation, caractérisée en ce que l'acide gras comprend de 6 à 30 atomes de carbone ou un dérivé d'un tel acide gras choisi parmi les esters, thioesters ou amides. De manière particulière, l'acide gras comprend de 10 à 22 atomes de carbone.

Selon un huitième mode de réalisation, la présente invention vise une composition selon le sixième ou septième mode de réalisation, caractérisée en ce que l'acide gras est choisi dans le groupe constitué par l'acide laurique, l'acide linoléique, l'acide linolénique, l'acide palmitique, l'acide pentadécanoique, l'acide caprique, l'acide oleique, l'acide myristique, l'acide palmitoléique, l'acide azélique, l'acide pimélique et leurs mélanges.

Dans un neuvième mode de réalisation, l'invention concerne une composition selon l'un des modes de réalisation 6 à 8, caractérisée en ce que la quantité de composé (1) est comprise en 0.1 et 5 % en poids de la composition.

Selon un dixième mode de réalisation, la présente invention concerne une composition selon l'un des modes de réalisation 7 à 9, caractérisée en ce que la quantité d'acide gras ou dérivé d'acide gras est comprise entre 95 et 99.9% en poids de la composition.

La présente invention concerne aussi, dans un onzième mode de réalisation, une formulation comprenant une composition selon l'un des modes de réalisation 7 à 10 et un solvant.

C'est aussi un douzième mode de réalisation de la présente invention que de fournir une formulation selon le onzième mode de réalisation, caractérisée en ce que le solvant est choisi dans le groupe constitué par l'eau, les alcools, les glycols, les polyglycols, la paraffine et leurs mélanges.

Dans un treizième mode de réalisation, l'invention vise une formulation selon le douzième mode de réalisation, caractérisée en ce que le solvant est choisi dans le groupe constitué par l'eau, l'éthanol ; l'isopropanol, la paraffine, le méthyl éther de dipropylène glycol, le propyl éther de propylène glycol, le méthyl éther de tri propylène glycol et leurs mélanges.

Dans une quatorzième mode de réalisation, la présente invention vise une composition selon l'un des modes de réalisation 6 à 13, pour son utilisation en tant que médicament vétérinaire pour le traitement de l'anxiété, le stress ou l'agressivité chez un mammifère non humain.

C'est un quinzième mode de réalisation de la présente invention que de fournir une composition selon le quatorzième mode de réalisation caractérisée en ce que le mammifère non humain est choisi dans le groupe comprenant les porcins, les ovins, les équidés, les bovins, les félins et les canidés.

Dans un mode de réalisation particulier, le mammifère non humain est le porc.

Dans un mode de réalisation particulier, le mammifère non humain est le chien.

Dans un mode de réalisation particulier, le mammifère non humain est le chat.

Dans un mode de réalisation particulier, le mammifère non humain est la vache.

La présente invention concerne aussi dans un seizième mode de réalisation, une formulation selon l'un des modes de réalisation 11 à 13 pour son utilisation en tant que médicament vétérinaire pour le traitement de l'anxiété, le stress ou l'agressivité chez un mammifère non humain.

Dans un dix-septième mode de réalisation, la présente invention vise une formulation selon le seizième mode de réalisation, caractérisée en ce que mammifère non humain est choisi dans le groupe comprenant les porcins, les ovins, les équidés, les bovins, les félins et les canidés.

Dans un mode de réalisation particulier, le mammifère non humain est le porc.

Dans un mode de réalisation particulier, le mammifère non humain est le chien.

Dans un mode de réalisation particulier, le mammifère non humain est le chat.

Dans un mode de réalisation particulier, le mammifère non humain est la vache.

Dans un dix-huitième mode de réalisation, l'invention concerne un dispositif d'administration à un mammifère non humain d'une composition selon l'un des modes de réalisation 6 à 10 ou d'une formulation selon l'un des mode de réalisation 11 à 13, en ce qu'il comprend un moyen de diffusion de ladite composition ou de ladite formulation pour permettre la diffusion de ladite composition ou de ladite formulation pendant une période allant de 1 à 35 jours.

Dans un dix-neuvième mode de réalisation de la présente invention, est aussi fourni un dispositif selon le dix-huitième mode de réalisation, caractérisé en ce qu'il est choisi dans le groupe constitué par un vaporisateur contenant ladite composition ou ladite formulation, un diffuseur électrique équipé d'une mèche et contenant ladite composition ou ladite formulation, un accessoire destiné à être porté par le mammifère non humain et imprégné de ladite composition ou de ladite formulation.

Enfin, la présente invention vise dans un vingtième mode de réalisation, un kit comprenant une composition selon l'un des modes de réalisation 6 à 10, une formulation selon l'un des modes de réalisation 11 à 13 et/ou un dispositif selon le mode de réalisation 18 ou 19, et une notice d'utilisation, destiné à diminuer le stress, l'agressivité et/ou l'anxiété d'un mammifère non humain.

Dans un mode de réalisation particulier, le mammifère non humain est le porc.

Dans un mode de réalisation particulier, le mammifère non humain est le chien.

Dans un mode de réalisation particulier, le mammifère non humain est le chat.

Dans un mode de réalisation particulier, le mammifère non humain est la vache.

Dans le cadre de la présente invention, le groupe R alkyl se réfère à une chaîne aliphatique linéaire, comportant de 2 à 30 atomes de carbone, particulièrement de 10 à 30 atomes de carbone, plus particulièrement encore de 12 à 25 atomes de carbone. Ce terme alkyl inclut des chaînes aliphatiques saturées ou insaturées, particulièrement monoinsaturées et di-insaturées. Les chaînes saturées préférées comprennent des chaînes C8, C10, C12, C14, C16, C18 ou C20 par exemple. Les chaînes insaturées comprennent les chaînes C16 :1, C18 :1, C22 :1 par exemple.

Comme évoqué, les dérivés d'acides gras selon l'invention peuvent comprendre des esters d'acide gras, c'est-à-dire un acide gras dont la fonction acide carboxylique est engagée avec une fonction alcool pour forme une liaison ester, c'est-à-dire encore le résultat de l'estérification du groupement carboxyl -COOH d'un acide gras avec un composé hydroxylé Les esters d'acide gras selon l'invention peuvent être choisis parmi les ester d'acide gras avec des alcools tels que methanol, éthanol, propanol, butanol par exemple.

Les dérivés d'acides gras selon la présente invention peuvent aussi comprendre des thioesters d'acides gras, c'est-à-dire un acide gras dont la fonction acide carboxylique est engagée avec la fonction thiol pour former une liaison thiester.

Les dérivés d'acides gras selon l'invention peuvent aussi comprendre des amides d'acides gras qui comprennent des acides gras dont le groupement carboxyl -COOH a été substitué par un groupement amide -CONH₂.

Par ailleurs, comme décrit précédemment la plupart des compositions apaisantes pour mammifères comportent des molécules présentes dans le lait maternel ou bien sécrétées par les glandes mammaires : acides gras, 2-méthyl-2-buténal, etc. Un autre type de molécules est présent dans le lait maternel : les éthers de glycérol plus communément appelés alkyl glycérol ou AKG et que l'on retrouve dans l'huile de foie de requin. Ces composés sont utilisés en médecine traditionnelle dans les pays nordiques. Ils sont connus pour leur capacité à améliorer les défenses immunitaires. Les laits maternels notamment humain, de vache, de brebis par exemple contiennent de nombreux AKG. En pourcentage, les plus importants sont AKG C16:0 ; AKG C18:0 ; AKG C18:1. Dans le lait humain apparaissent aussi d'autres AKG en quantité notable AKG C22:1 et AKG C24:1. Les distributions d'AKG dépendent donc des espèces ce qui rend cette famille spécifique de chaque espèce.

Pour former le composé (1), objet de la présente invention la demanderesse s'est donc en particulier intéressée aux radicaux R tels que, lorsque le 2-méthyl-2-buténal est relargué en présence d'un catalyseur acide, la molécule ainsi formée par clivage soit aussi intéressante pour l'application de la présente invention, c'est-à-dire l'apaisement des mammifères non humains. Cette deuxième molécule peut par exemple être un des AKG correspondant à ceux présents dans le lait maternel tels que cités ci-dessus par exemple ou un des esters gras de glycerol (monoglycéride), eux aussi présents dans le lait par exemple.

Dans un mode de réalisation de la présente invention est aussi fourni, un procédé de préparation d'un composé (1) selon la présente invention, en particulier selon l'un des modes de réalisation 1 à 3, selon le schéma 1 en faisant réagir un AKG ou un monoglycéride comportant un radical R, avec le 2-méthyl-2buténal, en milieu anhydre avec un pyridinium suivi d'un lavage basique comme indiqué ci-dessous.

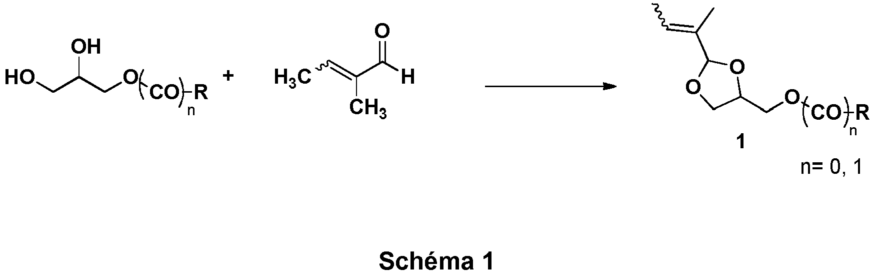

Dans lequel n est égal à 0 ou 1 et R représente un groupe alkyl linéaire saturé ou insaturé comportant de 2 à 30 atomes de carbone.

Dans un mode de réalisation, n=0 et R est un groupe alkyl linéaire saturé ou insaturé comportant entre 9 et 30 atomes de carbone, plus particulièrement entre 12 et 30 atomes de carbone.

Dans un autre mode de réalisation n=1 et R est un groupe alkyl linéaire saturé ou insaturé comportant de 2 à 30 atomes de carbone, plus particulièrement de 8 à 22 atomes de carbone.

### COMPOSITIONS SUIVANT l'INVENTION

Les compositions actives selon l'invention sont des compositions pro-phéromonales à base de composés nouveaux de formule générale (1) et d'acides gras ou de leurs dérivés. Le terme de pro-phéromonal signifie que la composition libère des molécules connues pour leur action phéromonale.

Les compositions selon l'invention sont formées d'un mélange d'un composé (1), qui représente entre 0.1% et 5% en poids de la composition totale, de préférence 1% en poids et d'un ou plusieurs acides gras ayant un nombre d'atomes de carbone compris entre 6 et 30 ou de leurs dérivés qui représentent au moins 85% en poids, voire 90% en poids , de préférence entre 99.5% et 90% en poids, de la composition.

Comme évoqué, la présente invention concerne une composition vétérinaire, comprenant un composé (1) selon la présente invention et au moins un acide gras ou dérive d'acide gras. La présence d'au moins un acide gras dans une composition selon l'invention permet de catalyser la décomposition lente du composé (1) en 2-méthyl-2-buténal et en AKG ou monoglyceride ayant le radical R.

Les compositions et formulations selon l'invention tiennent compte de la forte volatilité du 2-méthyl-2-buténal (point d'ébullition < 100°C) et de la faible volatilité des acides gras (point d'ébullition >180-200°C). En effet, l'acidité faible et le pKa proche de 5 des acides gras permet une hydrolyse lente du composé (1), qui va ainsi libérer du 2-méthyl-2-buténal. Cet aldéhyde va ainsi s'évaporer rapidement du fait de la forte volatilité et l'équilibre de l'équation chimique du schéma 1 va se déplacer vers la formation accrue de celui-ci, ce qui a pour conséquence une libération progressive du 2-méthyl-2-buténal. La mise en oeuvre de température élevée permet de faciliter l'évaporation du 2-méthyl-2-buténal qui accélère la réaction d'hydrolyse par déplacement de l'équilibre. C'est ainsi ce qui se passe lorsqu'une composition ou une formulation selon l'invention est mise en contact d'un dispositif de diffusion chauffant à des températures de l'ordre de 70 à 110°C tels que des flacons munis de mèche poreuse en contact avec un corps chauffant ou lorsque des plaquettes en cellulose imprégnées d'une composition ou formulation selon l'invention sont mise en contact avec la partie chauffante de diffuseurs électriques. Dans un mode alternatif de diffusion, une formulation selon l'invention peut être vaporisée dans l'air ou sur un support. Dans ces conditions, le solvant s'évapore rapidement et par conséquent, le mélange composé (1) et acide gras se trouve concentré. Dès lors le 2-méthyl-2-buténal dont la libération est initiée via l'hydrolyse du composé (1) via la présence de l'acide gras va s'évaporer facilement du fait de l'importante surface de contact ce qui va déséquilibrer l'équilibre réaction vers la génération de 2-méthyl-2-buténal. Les acides gras bien que moins volatiles seront malgré tout perçus par l'animal. Dans une situation dans laquelle le système de diffusion est un dispositif imprégné par la composition ou formulation selon l'invention, tel que par exemple un collier ou une boucle d'oreille, c'est la chaleur corporelle de l'animal permet d'atteindre des températures de l'ordre de 40 °C au niveau dudit dispositif qui va permettre d'initier la diffusion du 2-méthyl-2-buténal et de déclencher la libération progressive de ce composé et des acides gras.

Lorsque qu'une composition, formulation ou dispositif selon l'invention est emballé dans une contenant clos, une faible partie de 2-méthyl-2-buténal est générée par l'hydrolyse acide mais du fait de la non évaporation dudit 2-méthyl-2-buténal, l'équilibre réactionnel est maintenu et le composé (1) ne se décompose pas ou seulement à une vitesse négligeable sur la durée de stockage dudit dispositif.

Par l'expression « dérivé d'acide gras », on entend ici un ester d'acide gras, un thioester d'acide gras ou un amide d'acide gras tel que défini plus avant.

Ainsi la composition selon l'invention pourra comprendre au moins un acide gras choisi dans le groupe constitué par l'acide laurique, l'acide linoléique, l'acide linolénique, l'acide palmitique, l'acide pentadécanoique, l'acide caprique, l'acide oleique, l'acide myristique, l'acide palmitoléique, l'acide azélique, l'acide pimélique et leurs mélanges.

Un autre objet de l'invention est l'utilisation de de compositions vétérinaires selon l'invention pures ou en dilution dans un solvant ou dans un matériau diffusant pour apaiser des mammifères comme les ongulés (porcins, bovins, caprins, ovins), les équidés, les espèces canines, les félins. De préférence les formulations actives diluées seront diffusées au moyen de vaporisateurs, de diffuseurs thermiques ou encore de colliers.

La présente invention concerne ainsi une formulation qui contient une composition selon l'invention et un solvant.

Le solvant d'une formulation selon l'invention peut être choisi dans le groupe constitué par l'eau, les alcools, les glycols, les polyglycols, la paraffine et leurs mélanges . Le solvant peut être particulièrement l'isopropanol, l'éthanol, le mélange eau/éthanol, la paraffine ; l'isopraffine, le méthyl éther de dipropylène glycol, le propyl éther de propylène glycol ou le méthyl éther de tripropylène glycol, ou leurs mélanges, par exemple

La quantité de solvant dans une formulation selon l'invention pourra être comprise entre 80 et 99.5 % en poids de la formulation, plus particulièrement entre 85 et 99 % en poids de la formulation, plus particulièrement encore entre 90 et 99% en poids de la formulation.

Un autre objet de l'invention consiste à utiliser de telles formulations pour améliorer le rendement des élevages porcins, ovins, caprins ou bovins.

Une formulation selon l'invention pourra comprendre une composition selon la présente invention en des quantités de l'ordre de 0,5 à 20% en poids de la formulation, particulièrement de 1 à 15% en poids de la formulation, plus particulièrement encore de 1 à 10 % en poids de la formulation.

Les compositions et formulations selon l'invention sont susceptibles d'être diffusées dans l'air ambiant à l'aide de tous moyens ou dispositifs de vaporisation, atomisation, nébulisation ou génération d'aérosols ou encore de diffusion par la chaleur cinétique tel que précédemment décrite. Les dispositifs chauffants ou non, susceptibles de permettre la diffusion dans l'air ambiant des acides gras et du 2-méthyl-2-buténal selon l'invention peuvent comprendre à titre d'exemples des aérosols, des nébulisateurs pneumatique ou à ultrasons, des atomiseur à ultrasons, ou des sources de chaleur par combustion telles qu'une flamme de bougie, un bec benzène, un réchaud à gaz, un bois à combustion lente, ou un atomiseur piézoélectrique ou une source de chaleur par contact, telle qu'une résistance électrique, un bain liquide d'eau ou d'huile, ou encore une source de chaleur solaire, telle qu'un dispositif optique qui concentre les rayons lumineux. De préférence, la source de chaleur choisie permet d'atteindre une température comprise entre 70 et 120°C pendant 1 à 35 jours.

A température ambiante, les compositions et formulations selon l'invention peuvent se présenter sous forme liquide ou sous forme solide, en fonction de la longueur et la structure de la chaine carbonée R ainsi que celle des acides gras mais aussi en fonction sur solvant.

Lorsque les compositions ou formulations selon l'invention se présentent sous forme liquide, elles pourront être diffusées via un vaporisateur afin de déposer une quantité adéquate en un point précis et donné, tel que le panier ou la couche du mammifère non humain qu'il est souhaitable d'apaiser. Le solvant va alors rapidement s'évaporer et laisser subsister le mélange composé (1) et acide gras ou dérivé d'acide gras. L'évaporation du 2-méthyl-2-buténal va modifier l'équilibre réactionnel et la libération de celui-ci se fera de manière lente et progressive.

Lorsque les compositions ou formulations présentent sous forme solide, elles peuvent être placées à proximité d'une source de chaleur telle que précédemment décrite. Lorsque les compositions ou formulations selon l'invention sont en phase huileuse liquide, ils sont absorbés sur un moyen de diffusion, tel qu'un support poreux qui est chauffé afin d'assurer la diffusion des mélanges décrits ci-dessus. Ces supports poreux pourront être choisis en fonction de leur nature chimique de façon à ce qu'il n'y ait pas d'interaction chimique, en particulier de dégradation des acides gras des compositions ou formulations décrites ci-dessus. A titre d'exemples de supports poreux susceptibles d'absorber une quantité efficace d'une composition ou formulation selon la présente invention, on peut citer les matrices polymériques naturelles ou synthétiques, par exemple constituées de PVC, ou de latex, les matrices minérales cristallines ou amorphes, constituées par exemple de céramique ou de pierre ponce, les matrices organiques par exemple en bois, en fibres végétales telles que les fibres végétales de coton ou de bambou.

Les supports poreux peuvent être entourés d'une enveloppe hermétique, avant leur mise en oeuvre, possédant une ouverture permettant de contrôler la cinétique de diffusion. L'enveloppe pourra être choisie pour sa compatibilité chimique avec les acides gras en contact avec le film interne, sa caractéristique d'étanchéité et sa capacité à conduire la chaleur de façon homogène. A titre d'exemples, le matériau qui constitue l'enveloppe peut être un plastique constitué de plusieurs couches de polymères de natures différentes, ou bien un film métallique, par exemple d'aluminium, ou encore une superposition de couches de films de natures différentes, par exemple de plastique, des films organiques, ou des films métalliques. Dans le cadre d'une composition ou formulation à l'état liquide, celles-ci pourront être conservées et stockées dans un flacon clos hermétiquement.

Dans un cas particulier, le support poreux ou non poreux pourra être un accessoire porté par le mammifère, en particulier un collier, une boucle d'oreille, par exemple, en matériau plastique imprégné par une composition ou formulation selon l'invention en une quantité permettant sa diffusion selon la cinétique évoquée plus avant, et ce grâce à la chaleur corporelle de l'animal portant ledit accessoire.

Les compositions et formulations selon l'invention sont particulièrement adaptées pour traiter l'anxiété, le stress ou l'agressivité chez le chat, ou chez le chien ou chez le porc ou chez la vache.

### Exemples

### Exemple 1 : SYNTHESE d'un composé selon l'invention dans lequel R est C₂₂H₄₃ avec n=0.

Dans un ballon tricol de 250 mL sous atmosphére d'azote, est introduit de *cis-*13-docosénol (15.0 g ; 46.21 mmol) et la triéthylamine (9.6 mL ; 69 mmol) dissous dans 50 mL de méthyltétrahydrofurane. Le milieu réactionnel est refroit à 0°C puis le chlorure de mésyle (3.9 mL ; 50.80 mmol) en solution dans 25 mL de méthyltétrahydrofurane. Le milieu réactionnel est alors laissé remonter à l'ambiante et agité pendant 90 minutes. 100 mL d'eau est alors introduit. La phase organique est alors lavée avec 100 mL d'une solution aqueuse d'HCl (0.1N), 100 mL d'eau (émulsion), 100 ml d'une solution de saumure, séchée sur MgSO₄, filtrée et concentrée sous vide. Un huile incolore qui cristallise à température ambiante est alors obtenue (16.1 g ; 86%). Le brut réactionnel est utilisé tel quel dans l'étape suivante de condensation avec le Solketal (cf RMN ¹H).

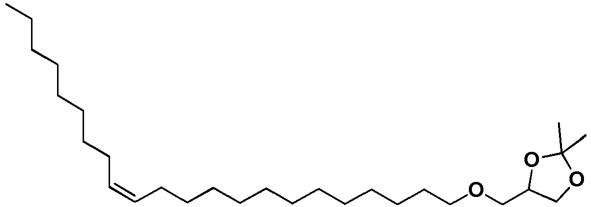

Dans un ballon tricol de 500 mL muni d'un Dean-Starck et sous atmosphère d'azote, est introduire le KOH finement broyé en suspension dans le toluène (110 mL). Le solketal (4.3 mL ; 34.7 mmol) et le dérivé mésylate préalablement synthétisé (15.9 g ; 39.50 mmol) sont introduits. Le milieu est alors chauffé au reflux pendant 16 heures. La réaction est alors quenchée par addition de 200 mL d'eau puis 100 mL d'acetate d'éthyle sont ajoutés de façon à avoir deux phases limpides. La phase aqueuse trouble est alors extraite de nouveau avec 2*100 mL d'acétate d'éthyle, lavée avec de la saumure (100 mL), séchée sur MgSO₄, filtrée et concentrée sous vide pour conduire au produit attendu sous la forme d'une huile orange (16.0 g ; 93%).

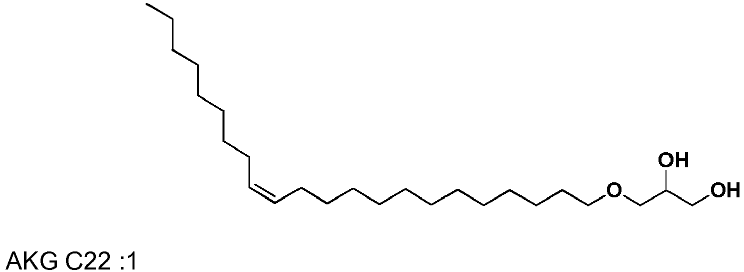

Dans un ballon monocol de 500 mL sous atmosphère d'azote, le dérivé dioxolane (15.80 g ; 36.0 mmol) est dissous dans un mélange méthanol/H₂O (158/15 mL). L'APTS est additionné (300 mg ; 1.74 mmol) est alors additionné au milieu réactionnel qui est chauffé pendant 3 heures à reflux. Le milieu est refroidi puis le méthanol est évaporé sous vide. Le résidu est alors repris dans 100 ml d'acétate d'éthyle, lavé avec une solution aqueuse saturée en NaHCO₃, avec 100 mL d'eau, avec de la saumure (100 mL), séché sur MgSO₄, filtré et concentré sous vide pour conduire à une huile qui cristallise à température ambiante. Le produit est cristallisé dans l'hexane (50 mL) pour conduire à un solide blanc (9.0 g ; 63%).

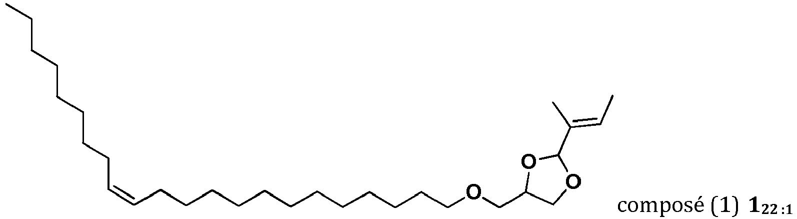

Dans un ballon tricol de 50 mL sous atmosphère d'azote, est introduit le dérivé alkylglycérol C22 : 1 (7 g ; 17.00 mmol) et le pyridinium paratoluènesulfonate (43 mg ; 0.17 mmol) en solution dans le toluène (90 mL). L'aldéhyde tiglique (11.5 mL ; 0118 mmol) en solution dans 10 mL de toluène est additionnée au milieu réactionnel qui est alors porté 3 heures au reflux. Le milieu réactionnel est neutralisé par addition d'une solution de NaHCO₃ saturée (50 mL). Après décantation, la phase aqueuse est extraite à l'acétate d'éthyle (50 mL).

Les phases organiques sont alors réunies, lavées avec une solution de saumure, séchées sur MgSO₄, filtréés et concentrées sous vide pour conduire à une huile jaune clair (8.0 g). Cette huile est alors purifiée par flash chromatographie pour conduire à une huile incolore (6.0 ; 74%).

Le tableau ci-dessous mentionne les différents alkylglycérol synthétisés et échantillonnés, ainsi que les conditions opératoires et les rendements globaux. Dans la partie expérimentale figurent les modes opératoires utilisés pour la synthèse de l'AKG C22 :1.

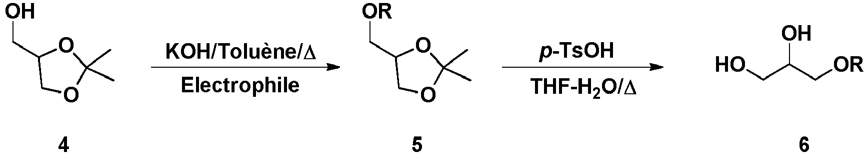

| R | AKG | Conditions | Groupe partant de l'électrophile | Produit **1** formé | Rendement (% mol.) |
|---|---|---|---|---|---|
| C₈H₁₇ | C_{8:0} | A | Iode | **1₈** | 37 |
| C₁₀H₂₁ | C_{10:0} | B | Brome | **1₁₀** | 68 |
| C₁₂H₂₅ | C_{12:0} | B | Brome | **1₁₂** | 62 |
| C₁₄H₂₉ | C_{14:0} | B | Brome | **1₁₄** | 65 |
| C₁₆H₃₃ | C_{16:0} | B | Mésylate | **1₁₆** | 66 |
| C₁₆H₃₁ | C_{16:1} | C | Mésylate | **1_{16:1}** | 66 |
| C₁₈H₃₇ | C_{18:0} | B | Mésylate | **1₁₈** | 65 |
| C₁₈H₃₅ | C_{18:1} | C | Mésylate | **1_{18:1}** | 66 |
| C₁₈H₃₅ | C_{18:2} | C | Mésylate | **1_{18:2}** | 64 |
| C₂₀H₄₁ | C_{20:0} | B | Brome | **1₂₀** | 71 |
| C₂₂H₄₃ | C_{22:1} | C | Mésylate | **1_{22:1}** | 50 |

| | | | | | |
|---|---|---|---|---|---|
| Conditions A : 1) Solkétal/KOH/RI/DMSO/50°C 2) APTS/MeOH/H₂O. Conditions B : 1) Solkétal/KOH/RBr/Toluène/DS/2) APTS/MeOH/H₂O. Conditions C : 1) CH₂Cl₂/Et₃N/MsCI 2) Solkétal/KOH/ROMs/Toluène/DS/Δ 3) APTS/MeOH/H₂O. | | | | | |

### Méthode GC utilisée :

| | |
|---|---|
| **Equipement:** | GC, Hewlett Packard 5890 Series II |
| **Détecteur** | FID |
| **Colonne:** | *HP5 30m, 0.53 mm, 0.88 µm* |
| **Pression:** | 11 psi |
| **Volume injecté:** | 1 µl |
| **Préparation d'échantillon :** | 4 mg/ml dans AcOEt |
| **Température injecteur :** | 270°C |
| **Température détecteur** : | 280°C |
| **Four :** | |

| | |
|---|---|
| **Température initiale** | 170°C |
| **Temps initial** | 3 min |
| **Gradient** | 5°C/min |
| **Température finale** | 260°C |
| **Temps final** | 10 min |
| **Run** | 31 min |

### Exemple 2 : Préparation de formulations actives apaisantes

Les formulations apaisantes sont réalisées dans un ordre précis puisque le principe de l'invention consiste à combiner un composé **(1)** avec un acide gras catalyseur de sa dégradation. Le procédé consiste donc à introduire dans un mélangeur de type réacteur agité, le solvant de formulation, l'acide gras ou le mélange d'acides gras. On laisse sous agitation pendant 1 heure le temps que le milieu soit parfaitement homogène puis on ajoute le composé (1) et on maintient pendant encore 1 heure sous agitation.

Le tableau suivant liste les mélanges qui ont été réalisés

| Exemple | Solvant (v/v) | Quantité solvant (g) | Acides gras | Quantité d'acide gras (g) | Composé (1) | Quantité de composé **(1)** (g) |
|---|---|---|---|---|---|---|
| 2a | Ethanol/eau 90/10 | 85 | Linoléique/ Pentadécanoique 95/5 | 12 | **1₁₈** | 3 |
| 2b | Isopropanol | 85 | Linoléique/ Pentadécanoique 95/5 | 12 | **1₁₈** | 3 |
| 2c | Paraffine IsoparV | 90 | Linoléique | 9.7 | **1_{22:1}** | 0.3 |
| 2d | Paraffine IsoparV | 95 | Linoléique | 4.85 | **1_{22:1}** | 0.15 |
| 2e | Paraffine IsoparV | 98 | Linoléique | 1.94 | **1_{22:1}** | 0.06 |
| 2f | Methyl éther de dipropylène glycol | 99 | Linoléique | 0.9 | **1_{22:1}** | 0.09 |
| 2g | propyl éther de proprylène glycol | 99 | Linoléique | 0.9 | **1_{22:1}** | 0.09 |
| 2h | méthyl éther de tri proprylène glycol | 99 | Linoléique | 0.9 | **1_{22:1}** | 0.09 |

### Exemple 3 : Etude in vitro du relargage de 2M2B

On utilise la solution préparée dans l'exemple 2b dans une enceinte climatique à 100°C et 30°C (conditions correspondant soit à la température d'un diffuseur thermique soit à une température ambiante). Des échantillons sont prélevés à intervalles réguliers et on mesure par chromatographie phase gaz la disparition du composé **1_{22:1}** et l'apparition de l'AKG C_{22:1}. Les résultats sont reportés dans le tableau suivant (les % sont exprimés en ne considérant que l'intégration relative au composé **1₁₈** et de l'AKG C_{18:0}) :

| T= 100°C | 0 | 3h | 6h | 1j | 2j | 7j | 14j | 21j | 28j |
|---|---|---|---|---|---|---|---|---|---|
| %**1₁₈** | 100 | 52 | 25 | 2 | 0 | 0 | 0 | 0 | 0 |
| % AKG C_{18:0} | 0 | 48 | 75 | 98 | 100 | 100 | 100 | 100 | 100 |

| T= 30°C | 0 | 3h | 8h | 1j | 2j | 7j | 14j | 21j | 28j |
|---|---|---|---|---|---|---|---|---|---|
| %**1₁₈** | 100 | | | 100 | 100 | 100 | 99.5 | 99 | 99 |
| % AKG C_{18:0} | 0 | | | 0 | 0 | 0 | 0.5 | 1 | 1 |

Ce tableau montre que la dégradation du produit **1₁₈** est bien stimulée par la température élevée (type diffuseur électrique) mais que dans son état dilué à température ambiante la solution est suffisamment stable pour assurer le traitement pendant un mois (mois de 5% de perte sur la durée).

De la même façon, nous avons constaté que les solutions des exemples 2f, 2g, 2h présentaient des cinétiques de libération du *trans*-2-méthyl-2-buténal similaires et ce malgré une nature différente du solvant de dilution.

### Exemple 4 : Formulation pour diffuseur thermique et courbe de relargage

On utilise 50 ml des formulations fabriquées dans les exemples 2c,d,e pour remplir trois flacon de PET équipés de mèche de diffusion. Chaque flacon est vissé sur un diffuseur électrique caractérisé en ce que la céramique qui le compose est chauffée à 100°C environ lorsque le système est branché.

On mesure ensuite le relargage journalier de formulation et les résultats sont reportés dans le tableau suivant : Est évaluée la perte de poids total de la formulation reflétant la libération du 2-méthyl-2-buténal, de l'acide gras ainsi que du solvant réunis.

| Jours | 1 | 2 | 3 | 4 | 5 | 6 | 7 | 14 | 21 | 28 | 35 | 42 | 49 | 56 |
|---|---|---|---|---|---|---|---|---|---|---|---|---|---|---|
| Formule | | | | | | | | | | | | | | |
| 2c | 1 | 0.5 | 0.3 | 0.3 | 0.2 | 0.2 | 0.1 | 0.1 | 0.05 | 0.05 | - | - | - | - |
| 2d | 0.9 | 1 | 0.8 | 0.6 | 0.4 | 0.2 | 0.2 | 0.15 | 0.15 | 0.15 | 0.15 | 0.15 | 0.15 | 0.15 |
| 2^{e} | 0.9 | 1.2 | 1.2 | 1.2 | 0.7 | 0.8 | 0.7 | 0.4 | 0.4 | 0.3 | 0.3 | 0.3 | 0.3 | 0.3 |

Le tableau exprime la diffusion journalière en gramme de chaque formulation

Au trente-cinquième jour, les compositions résiduelles dans chaque flacon sont mesurées par chromatographie liquide et comparées aux compositions initiales.

| Formule | Composition initiale | Composition après 35 j de diffusion |
|---|---|---|
| 2c | 90/9.7/0.3 | 88/11.6/0.1 |
| 2d | 95/4.85/0.15 | 95/4.95/0.15 |
| 2e | 98/1.94/0.06 | 98/1.94/0.06 |

Le tableau exprime la teneur de chaque solution selon le ratio Paraffine/Acide linoléique/**1_{22:1}**

Suite aux mesures précédentes, nous pouvons estimer que les doses relarguées sur la période de 35 jours par molécules entrant dans chaque composition sont les suivantes (en gramme) :

| Formule | 2c | 2d | 2e |
|---|---|---|---|
| Acide linoléique | 0.195 | 0.188 | 0.2 |
| **1_{22:1}** | 0.05 | 0.0063 | 0.0063 |

Ces résultats démontrent que le produit **1_{22:1}** et l'acide linoléique disparaissent de la formulation à des débits qui respectent les ordres de grandeur définis dans la formulation.

En considérant qu'à 100°C, tout le produit **1_{22:1}** se décompose comme cela a été montré dans l'exemple 3, nous pouvons estimer les doses journalières des différents produits relargués dans l'atmosphère selon les différentes formules.

### Formule à 2% (2e)

| Produit | Dose journalière (mg) |
|---|---|
| Acide linoléique | 5.8 |
| AKG C_{22:1} | 0.14 |
| 2-Méthyl-2-buténal | 0.036 |

### Formule à 5% (2d)

| Produit | Dose journalière (g) |
|---|---|
| Acide linoléique | 5.1 |
| AKG C_{22:1} | 0.14 |
| 2-Méthyl-2-buténal | 0.036 |

### Formule à 10% (2c)

| Produit | Dose journalière (mg) |
|---|---|
| Acide linoléique | 5.5 |
| AKG C_{22:1} | 1.2 |
| 2-Méthyl-2-buténal | 0.32 |

### Exemple 6 : Etude de l'effet apaisant sur cochon

Les tests ont été conduits avec des diffuseurs électriques comme ceux décrits dans l'exemple 5.

Le protocole est le suivant :
100 porcelets de 3 semaines sont répartis dans 4 porcheries sur caillebotis de 25m2 (soit 175 m3). Les porcelets sont répartis par populations de 25 appelées A,B,C ,D. La population A est traitée avec un diffuseur ne contenant que de la paraffine isopar V ;
La population B est traitée par un diffuseur contenant la formule de l'exemple 2e (2%).

La population B est traitée par un diffuseur contenant la formule de l'exemple 2d (5%).

La population B est traitée par un diffuseur contenant la formule de l'exemple 2c (10%).

Les tests sont conduits pendant 28 jours. On mesure chaque semaine les poids moyens de chaque population et le nombre de blessures apparentes moyennes.

Les résultats sont reports dans les tableaux suivants:

| Evolution des poids en fonction de l'âge des porcelets (kg) | A | B | C | D |
|---|---|---|---|---|
| Poids à 21 jours | 6.513 | 6.513 | 6.513 | 6.513 |
| Poids à 28 jours | 7.081 | 7.075 | 7.078 | 7.051 |
| Poids à 35 jours | 9.153 | 9.161 | 9.175 | 9.169 |
| Poids à 42 jours | 11.815 | 12.012 | 12.02 | 12.07 |
| Poids à 49 jours | 14.989 | 15.206 | 15.281 | 15.601 |

| Gains de poids moyens quotidiens (kg) | A | B | C | D |
|---|---|---|---|---|
| Semaine 1 | 0,08 | 0,08 | 0,08 | 0,08 |
| Semaine 2 | 0,30 | 0,30 | 0,30 | 0,30 |
| Semaine 3 | 0,38 | 0,41 | 0,41 | 0,41 |
| Semaine 4 | 0,45 | 0,46 | 0,47 | 0,50 |

Le nombre de morsure sur les quatre semaines de traitement est inférieur de 10 à 12% pour les populations B, C et D par rapport à la population A.

### Exemple 7 : Effet apaisant sur chaton

Un vaporisateur de 60 ml est rempli avec la formulation de l'exemple 2a. Un autre vaporisateur contenant un mélange méthanol/eau (90/10 v/v) utilisé en tant que placebo.

6 chatons de 1 mois d'une même portée sont séparés de leur mère et repartis en deux groupes de trois (2 mâles, 1 femelle dans chaque groupe). Le groupe A est placé dans un panier sur lequel on vaporise toutes les 12 h la formulation 2a. Le groupe B est placé dans un panier vaporisé toutes les 12 h avec le placébo .

Dans la première semaine les chatons de la population A sont calmes et dorment en moyenne 16 heures par jour les uns contre les autres tandis que les chatons de la population B dorment au plus 14 heures et au bout du troisième jour se disputent régulièrement.

La deuxième semaine les comportements des deux populations convergent.

Cet exemple montre que la formulation A en vaporisateur permet d'améliorer l'adaptation des chatons au sevrage.

## Revendications

1. Composé de formule générale (1) : 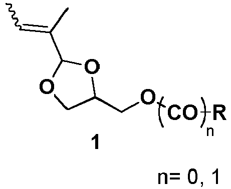 dans laquelle n est égal à 0 ou 1 et R représente un groupe alkyl linéaire saturé ou insaturé comportant de 2 à 30 atomes de carbone.

2. Composé selon la revendication 1, **caractérisé en ce que** n=0 et R est un groupe alkyl linéaire saturé ou insaturé comportant entre 9 et 30 atomes de carbone, plus particulièrement entre 12 et 30 atomes de carbone.

3. Composé selon la revendication 1, **caractérisé en ce que** n=1 et R est un groupe alkyl linéaire saturé ou insaturé comportant de 2 à 30 atomes de carbone, plus particulièrement de 8 à 22 atomes de carbone.

4. Composé selon l'une des revendications précédentes pour son utilisation en tant que médicament vétérinaire pour le traitement de l'anxiété, du stress ou de l'agressivité chez un mammifère non humain.

5. Composé pour son utilisation selon la revendication 4, **caractérisé en ce que** le mammifère non humain est choisi dans le groupe comprenant les porcins, les ovins, les équidés, les bovins, les félins et les canidés.

6. Composition vétérinaire comprenant un composé (1) selon l'une des revendications 1 à 3 et au moins un acide gras ou un de ses dérivés.

7. Composition selon la revendication 6, **caractérisée en ce que** l'acide est un acide gras ayant 6 à 30 atomes de carbone ou un dérivé d'un tel acide gras choisi parmi les esters, thioesters ou amides.

8. Composition selon la revendication 7, **caractérisée en ce que** l'acide gras est choisi dans le groupe constitué par l'acide laurique, l'acide linoléique, l'acide linolénique, l'acide palmitique, l'acide pentadécanoique, l'acide caprique, l'acide oleique, l'acide myristique, l'acide palmitoléique, l'acide azélique, l'acide pimélique et leurs mélanges.

9. Composition selon l'une des revendications 6 à 8, **caractérisée en ce que** la quantité de composé (1) est comprise en 0.1 et 5 % en poids de la composition.

10. Composition selon l'une des revendications 6 à 9, **caractérisée en ce que** la quantité d'acide gras ou dérivé d'acide gras est comprise entre 95 et 99.9% en poids de la composition.

11. Formulation comprenant une composition selon l'une des revendications 6 à 10 et un solvant.

12. Formulation selon la revendication 11, **caractérisée en ce que** le solvant est choisi dans le groupe constitué par l'eau, les alcools, les glycols, les polyglycols, la paraffine et leurs mélanges.

13. Formulation selon la revendication 12, **caractérisée en ce que** le solvant est choisi dans le groupe constitué par l'eau, l'éthanol, l'isopropanol, la paraffine, le méthyl éther de dipropylène glycol, le propyl éther de propylène glycol, le méthyl éther de tri propylène glycol et leurs mélanges.

14. Composition selon l'une des revendications 6 à 10, pour son utilisation en tant que médicament vétérinaire pour le traitement de l'anxiété, le stress ou l'agressivité chez un mammifère non humain.

15. Composition pour son utilisation selon la revendication 14, **caractérisé en ce que** le mammifère non humain est choisi dans le groupe comprenant les porcins, les ovins, les équidés, les bovins, les félins et les canidés.

16. Formulation selon l'une des revendications 11 à 13, pour son utilisation en tant que médicament vétérinaire pour le traitement de l'anxiété, le stress ou l'agressivité chez un mammifère non humain.

17. Formulation pour son utilisation selon la revendication 16, **caractérisée en ce que** mammifère non humain est choisi dans le groupe comprenant les porcins, les ovins, les équidés, les bovins, les félins et les canidés.

18. Dispositif d'administration à un mammifère non humain d'une composition selon l'une des revendications 6 à 10 ou d'une formulation selon l'une des revendications 11 à 13, **caractérisé en ce qu'**il est choisi dans le groupe constitué par : un vaporisateur contenant ladite composition ou ladite formulation, un diffuseur électrique équipé d'une mèche et contenant ladite composition ou ladite formulation, un accessoire destiné à être porté par le mammifère non humain et imprégné de ladite composition ou de ladite formulation.

19. Kit comprenant une composition selon l'une quelconque des revendications 6 à 10, une formulation selon l'une des revendications 11 à 13 et/ou un dispositif selon la revendication 18, destiné à diminuer le stress, l'agressivité et/ou l'anxiété d'un mammifère non humain.

## Patentansprüche

1. Verbindung mit der allgemeinen Formel (1): 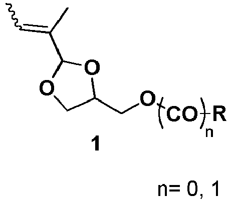 wobei n gleich 0 oder 1 ist und R eine gesättigte oder ungesättigte lineare Alkylgruppe mit 2 bis 30 Kohlenstoffatomen darstellt.

2. Verbindung nach Anspruch 1, **dadurch gekennzeichnet, dass** n = 0 und R eine gesättigte oder ungesättigte lineare Alkylgruppe ist, die zwischen 9 und 30 Kohlenstoffatome und insbesondere zwischen 12 und 30 Kohlenstoffatome umfasst.

3. Verbindung nach Anspruch 1 **dadurch gekennzeichnet, dass** n = 1 und R eine gesättigte oder ungesättigte lineare Alkylgruppe ist, die von 2 bis 30 Kohlenstoffatome, insbesondere von 8 bis 22 Kohlenstoffatome, umfasst.

4. Verbindung nach einem beliebigen der vorstehenden Ansprüche zur Verwendung als Tierarzneimittel zur Behandlung von Angst, Stress oder Aggressivität bei einem nicht-menschlichen Säugetier.

5. Verbindung zur Verwendung nach Anspruch 4, **dadurch gekennzeichnet, dass** das nicht menschliche Säugetier aus der Gruppe bestehend aus Schweinen, Schafen, Equiden, Rindern, Katzen und Caniden ausgewählt ist.

6. Tierärztliche Zusammensetzung, die eine Verbindung (1) nach einem beliebigen der vorstehenden Ansprüche 1 bis 3 und zumindest eine Fettsäure oder eines ihrer Derivate umfasst.

7. Zusammensetzung nach Anspruch 6, **dadurch gekennzeichnet, dass** die Säure eine Fettsäure mit 6 bis 30 Kohlenstoffatomen oder ein Derivat einer solchen Fettsäure ist, das unter Estern, Thioestern oder Amiden ausgewählt ist.

8. Zusammensetzung nach Anspruch 7, **dadurch gekennzeichnet, dass** die Fettsäure aus der Gruppe bestehend aus Laurinsäure, Linolsäure, Linolensäure, Palmitinsäure, Pentadecansäure, Caprinsäure, Ölsäure, Myristinsäure, Palmitoleinsäure, Azelsäure, Pimelinsäure und ihren Mischungen ausgewählt ist.

9. Zusammensetzung nach einem beliebigen der vorstehenden Ansprüche 6 bis 8, **dadurch gekennzeichnet, dass** die Menge der Verbindung (1) zwischen 0,1 und 5 Gew.-% der Zusammensetzung beträgt.

10. Zusammensetzung nach einem beliebigen der vorstehenden Ansprüche 6 bis 9, **dadurch gekennzeichnet, dass** die Menge an Fettsäure oder Fettsäurederivat zwischen 95 und 99,9 Gew.-% der Zusammensetzung liegt.

11. Formulierung, die eine Zusammensetzung nach einem beliebigen der vorstehenden Ansprüche 6 bis 10 und ein Lösungsmittel umfasst.

12. Formulierung nach Anspruch 11, **dadurch gekennzeichnet, dass** das Lösungsmittel aus der Gruppe bestehend aus Wasser, Alkoholen, Glykolen, Polyglykolen, Paraffin und ihren Mischungen ausgewählt ist.

13. Formulierung nach Anspruch 12, **dadurch gekennzeichnet, dass** das Lösungsmittel aus der Gruppe bestehend aus Wasser, Ethanol, Isopropanol, Paraffin, Dipropylenglykolmethylether, Propylenglykolpropylether, Propylenglykolmethylether und Mischungen davon ausgewählt ist.

14. Zusammensetzung nach einem beliebigen der vorstehenden Ansprüche 6 bis 10 zur Verwendung als Tierarzneimittel zur Behandlung von Angst, Stress oder Aggressivität bei einem nicht-menschlichen Säugetier.

15. Verbindung zur Verwendung nach Anspruch 14, **dadurch gekennzeichnet, dass** das nicht-menschliche Säugetier aus der Gruppe bestehend aus Schweinen, Schafen, Equiden, Rindern, Katzen und Caniden ausgewählt ist.

16. Formulierung nach einem der vorstehenden Ansprüche 11 bis 13 zur Verwendung als Tierarzneimittel zur Behandlung von Angst, Stress oder Aggressivität bei einem nicht-menschlichen Säugetier.

17. Formulierung zur Verwendung nach Anspruch 16, **dadurch gekennzeichnet, dass** das nicht menschliche Säugetier aus der Gruppe bestehend aus Schweinen, Schafen, Equiden, Rindern, Katzen und Caniden ausgewählt ist.

18. Vorrichtung zum Verabreichen einer Zusammensetzung nach einem beliebigen der vorstehenden Ansprüche 6 bis 10 oder einer Formulierung nach einem beliebigen der vorstehenden Ansprüche 11 bis 13 an ein nicht-menschliches Säugetier, **dadurch gekennzeichnet, dass** sie aus der Gruppe bestehend aus: einem Verdampfer, der die Zusammensetzung oder die Formulierung enthält, einem elektrischen Diffusor, der mit einem Docht ausgestattet ist und die Zusammensetzung oder die Formulierung enthält, einem Zubehör, das dazu bestimmt ist, von dem nicht-menschlichen Säugetier getragen und mit der Zusammensetzung oder der Formulierung imprägniert zu werden, ausgewählt ist.

19. Kit, umfassend eine Zusammensetzung nach einem beliebigen der vorstehenden Ansprüche 6 bis 10, eine Formulierung nach einem beliebigen der vorstehenden Ansprüche 11 bis 13 und/oder eine Vorrichtung nach Anspruch 18 zur Verringerung von Stress, Aggressivität und/oder Angst eines nicht-menschlichen Säugetiers.

## Claims

1. A compound of the general formula (1): 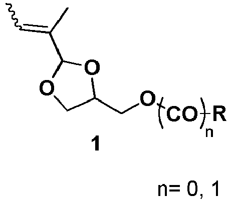 wherein n is equal to 0 or 1 and R represents a saturated or unsaturated linear alkyl group having 2 to 30 carbon atoms.

2. The compound as claimed in claim 1, **characterized in that** n = 0 and R is a saturated or unsaturated linear alkyl group having 9 to 30 carbon atoms, more particularly 12 to 30 carbon atoms.

3. The compound as claimed in claim 1, **characterized in that** n = 1 and R is a saturated or unsaturated linear alkyl group having 2 to 30 carbon atoms, more particularly 8 to 22 carbon atoms.

4. The compound as claimed in one of the preceding claims for use as a veterinary medicinal product for treating anxiety, stress or aggressiveness in a non-human mammal.

5. The compound for use as claimed in claim 4, **characterized in that** the non-human mammal is selected from the group consisting of pigs, sheep, horses, cattle, cats and dogs.

6. A veterinary composition comprising a compound (1) as claimed in one of claims 1 to 3 and at least one fatty acid or a derivative thereof.

7. The composition as claimed in claim 6, **characterized in that** the acid is a fatty acid having 6 to 30 carbon atoms or a derivative of such a fatty acid selected from esters, thioesters or amides.

8. The composition as claimed in claim 7, **characterized in that** the fatty acid is selected from the group consisting of lauric acid, linoleic acid, linolenic acid, palmitic acid, pentadecanoic acid, capric acid, oleic acid, myristic acid, palmitoleic acid, azelaic acid, pimelic acid and mixtures thereof.

9. The composition as claimed in one of claims 6 to 8, **characterized in that** the amount of the compound (1) is between 0.1% and 5% by weight of the composition.

10. The composition as claimed in one of claims 6 to 9, **characterized in that** the amount of fatty acid or fatty acid derivative is between 95% and 99.9% by weight of the composition.

11. A formulation comprising the composition as claimed in one of claims 6 to 10 and a solvent.

12. The formulation as claimed in claim 11, **characterized in that** the solvent is selected from the group consisting of water, alcohols, glycols, polyglycols, paraffin and mixtures thereof.

13. The formulation as claimed in claim 12, **characterized in that** the solvent is selected from the group consisting of water, ethanol, isopropanol, paraffin, dipropylene glycol methyl ether, propylene glycol propyl ether, tripropylene glycol methyl ether and mixtures thereof.

14. The composition as claimed in one of claims 6 to 10, for use as a veterinary medicinal product for treating anxiety, stress or aggressiveness in a non-human mammal.

15. The composition for use as claimed in claim 14, **characterized in that** the non-human mammal is selected from the group consisting of pigs, sheep, horses, cattle, cats and dogs.

16. The formulation as claimed in one of claims 11 to 13, for use as a veterinary medicinal product for treating anxiety, stress or aggressiveness in a non-human mammal.

17. The formulation for use as claimed in claim 16, **characterized in that** the non-human mammal is selected from the group consisting of pigs, sheep, horses, cattle, cats and dogs.

18. A device for administering to a non-human mammal the composition as claimed in one of claims 6 to 10 or the formulation as claimed in one of claims 11 to 13, **characterized in that** it is selected from the group consisting of: a vaporizer containing said composition or said formulation, an electric diffuser fitted with a wick and containing said composition or said formulation, an accessory designed to be carried by the non-human mammal and impregnated with said composition or said formulation.

19. A kit comprising the composition as claimed in any one of claims 6 to 10, the formulation as claimed in one of claims 11 to 13 and/or the device as claimed in claim 18, for decreasing stress, aggressiveness and/or anxiety in a non-human mammal.
